# EUROPEAN PATENT SPECIFICATION

(11) **EP 1 742 674 B1**
(45) Date of publication and mention of the grant of the patent: **30.12.2009**
(21) Application number: 05735124.9
(22) Date of filing: 26.04.2005
(51) Int. Cl.: A61L 2/26

(54) **DOOR DEVICE FOR DISINFECTION CHAMBER**
TÜRVORRICHTUNG FÜR EINE DESINFEKTIONSKAMMER
DISPOSITIF DE PORTE CONÇU POUR UNE ENCEINTE DE DESINFECTION

(30) Priority: 27.04.2004 SE 0401075
(43) Date of publication of application: 17.01.2007
(73) Proprietor: Getinge Disinfection AB, 351 15 Växjö (SE)
(72) Inventor: JÖNSSON, Christer, S-352 53 Växjö (SE)
(74) Representative: Fritsche, Daniel
(86) International application number: PCT/SE2005/000604
(87) International publication number: WO 2005/102400

(56) References cited:
- EP-A2- 0 259 009
- CH-A5- 688 940
- US-A- 3 386 203
- US-A- 5 195 790
- US-A- 5 566 508
- US-A- 6 017 105
- US-B1- 6 264 901
- US-B1- 6 416 144

## Description

### Field of the Invention

The present invention relates to a disinfection apparatus for disinfecting fluid cleaning of objects, such as health care objects, which disinfection apparatus comprises a housing which forms a chamber with an entrance, a substantially laterally displaceable door leaf which is arranged in the closed position to close said entrance, and at least one guide element which extends substantially in the direction of motion of the door leaf and in whose direction of extension the door leaf is displaceably arranged.

### Background Art

In medical case for instance, disinfection is an important activity to prevent the spread of infection and bacterial growth. There are today a large number of different disinfection apparatus, which are adapted to be installed, for instance, in hospital wards for disinfection of goods, instruments and other objects. As examples of such objects, mention can be made of vessels of different kinds, instrument containers, wheelchairs, trolley equipment, hospital beds, surgery equipment, instruments, animal cages, machine parts in nursing applications and other bulky objects.

One type of disinfection apparatus is provided with what is referred to as walk-in chambers, which are large enough for an individual to enter and/or large enough for a trolley/cart or other equipment to be inserted. As a rule such a disinfection apparatus comprises a disinfection chamber, into which nozzles open for supplying disinfection fluid. The nozzles are usually connected via a pump to a separate water tank which is supplied from a public water system. As a rule, the disinfection chamber is made of stainless steel and closed by a conventional door unit, such as a laterally operated door unit or a pivotally operated door unit comprising one or more door leaves. Laterally operated door units are usually slidingly guided along guides which are arranged at the upper edge and lower edge of the door unit to guide it between its open position and its closed position, and vice versa. Pivotally operated doors often require much space in their open position and can thus be an obstacle to the activity performed in connection with the use of the disinfection chamber as well as to other activities performed in connection with the disinfection chamber.

One example of prior art is D1, CH688940, which discloses a pressurized container, in particular sterilizer, which is sealed by a sliding door. The door is arranged to move horizontally towards an open position at a wedge angle to a maximum distance from and at the same level of the frame. Another prior art document is D2, US6017105 which discloses a guidance assembly which guides a door to a cabinet while it is moved between an open position and a closed position.

There are however a number of drawbacks in connection with the closing of the disinfection chambers that are currently available.

The available disinfection chambers frequently comprise a threshold or other elevations to obtain a reliable seal between the disinfection chamber and its surroundings. Such an elevation may, for instance, cause trouble when a trolley or like object is to be inserted into the disinfection chamber. Troubles of this type may imply that it takes long to insert and remove objects from the disinfection chamber. Moreover elevations in connection with the entrance to the chamber may cause a risk of careless handling of the objects that are to be disinfected. Elevations of the type mentioned may also result in impaired ergonomic conditions for the operator.

Some of the available disinfection chambers can be installed in a chamber cavity, which is also referred to as a pit, in the installation floor so that a water-permeable chamber floor and the surrounding floor outside the entrance to the chamber are positioned at the same level. For such a chamber it is possible to obtain a thresholdless entrance to the chamber by lowering the chamber door and the associated seal at the lower edge of the door below the level of the surrounding floor. Such an installation, however, will in many cases be expensive and require great resources while at the same time the room in which the disinfection apparatus is to be installed will have to be greatly modified. Great efforts are required in particular in connection with installations of disinfection chambers which are provided with a laterally displaceable door.

It also happens that a previously used disinfection apparatus is replaced, which often requires adaptations to the new apparatus by reshaping of said pit. Such adaptations often require both great resources and much time.

Disinfection chambers of the type described above have, as mentioned above, usually a liquid-permeable chamber floor mounted over a bottom of the chamber, on which chamber floor objects to be cleaned are intended to be placed for cleaning. When liquid cleaning of objects is performed in the chamber, the descending liquid is collected at the bottom of the chamber, the liquid flowing to a pump which makes it possible to pump out liquid from the liquid system or return liquid thereto. As a result of the collection of cleaning liquid at the bottom of the chamber, it is above all necessary to establish a reliable seal between the lower edge of the door of the disinfection apparatus and the corresponding lower part of the door post to prevent leakage from the disinfection chamber to the surroundings.

### Summary of the Invention

The object of the present invention is to provide a disinfection apparatus which allows improvements in relation to prior-art disinfection apparatus in one or more of the above aspects.

In one aspect of the present invention, a disinfection apparatus is provided for disinfecting fluid cleaning of objects, such as health care objects, which disinfection apparatus comprises a housing which forms a chamber with an entrance, a substantially laterally displaceable door leaf which is arranged in the closed position to close said entrance, and at least one guide element which extends substantially in the direction of motion of the door leaf and in whose direction of extension the door leaf is displaceably arranged, wherein the lower edge of the door leaf constitutes a door sealing portion which is arranged to sealingly cooperate with a corresponding lower frame sealing portion in the closed position, the door sealing portion of the door leaf being arranged to be moved vertically relative to said opposite frame sealing portion when the door leaf is moved from the closed position to an open position.

The invention according to claim 1 gives a number of advantages, such as that reduced efforts and resources for installation of disinfection chambers with laterally displaceable door leaves are made possible compared with prior-art technique.

Moreover the disinfection apparatus according to the invention makes it possible to replace old disinfection apparatus, which comprise door units of other types, by a disinfection apparatus according to the invention without the need for extra efforts for arranging a recess in the floor, which recess usually in prior-art technique is necessary to hold the displaceable door leaf as this is moved between the closed position and the open position, and vice versa. The disinfection apparatus according to the invention thus makes it possible to replace the frequently used pivotally operable door units by slidably operable door units, thereby reducing efforts and costs in relation to prior art for such a reconstruction.

The guide element of the disinfection apparatus is preferably at least partly arranged with an inclined extent α, in the direction of motion of the door leaf from the closed position to the open position, in relation to a horizontal line. The guide element thus allows the door leaf to be raised in the vertical direction when being moved from the closed to the open position, and the door leaf will thus be lowered as it is moved from the open position to the closed position. Consequently, the vertical movement of the door leaf will be substantially continuous during opening and closing of the door unit.

Said frame sealing portion is preferably arranged as a part of a threshold cavity. By arranging the frame sealing portion as a part of the threshold cavity, in which the door leaf is inserted at least in the closed position, a sealing cooperation is made possible between the side of the door leaf which is directed inwards to the disinfection chamber and the frame sealing portion. Such a seal also makes it possible to prevent water from entering the threshold cavity in operation of the disinfection apparatus.

According to one embodiment, said threshold cavity is preferably arranged in a groove in the floor surrounding the disinfection apparatus. By arranging the threshold cavity as a groove sunk into the floor, it is possible to obtain a disinfection apparatus with a thresholdless entrance to the chamber. By thresholdless is here meant that the entrance to the chamber is substantially at the same level as the floor surrounding the disinfection apparatus and the floor inside the chamber.

According to an alternative embodiment, said threshold cavity is arranged as a groove in at least a part of a threshold portion, which at least partly extends above the floor surrounding the disinfection apparatus.

In one embodiment, the disinfection apparatus is arranged so that the door leaf can be moved into sealing cooperation with the housing in the closed position by a substantially horizontal displacement of the door leaf inwards to said chamber of the disinfection apparatus. By making it possible to move the door leaf into sealing engagement with the housing of the disinfection apparatus and removing it therefrom, it is made possible to displace the door leaf in the lateral direction along the guide element without friction being exerted by seals that are arranged between the door leaf and the housing.

Said at least one guide element of the housing is arranged to extend above the door leaf, and the door leaf is slidingly engaged with the guide element by at least one sliding means. Moreover, said at least one sliding means is arranged as a roll element, which is preferably connected to the door leaf. According to a particularly preferred embodiment, said guide element is designed as an elongate square section with a slot arranged in the longitudinal direction thereof through a wall portion, said roll element comprising at least two rolls which are adapted to run one on each side of said slot inside the section. However, it will be appreciated by a person skilled in the art that the sliding means and the guide element can be designed in various ways in order to guide the door leaf between the open and the closed position. It will also be appreciated that it is possible to arrange the guide element as a part of the door leaf and the sliding means as a part of the housing, where the door leaf is moved from the closed position to the open position, and vice versa, by the guide element sliding on the sliding means.

According to a preferred embodiment, the door leaf is connected to a driving belt to be slidably moved between the closed and the open position, and vice versa, which driving belt is driven by a motor. Such a drive of the door leaf makes it possible to automate the opening and closing of the entrance to the disinfection apparatus.

The inclined extent α of the guide element preferably is an angle between 1° and 10°, more preferred an angle between 1° and 5°, and in particular an angle between 2° and 3°. The angle controls how quickly the door leaf will be moved in the vertical direction relative to the frame sealing portion, the angle being selected, inter alia, depending on the depth and length of the threshold cavity.

To allow the interior of the disinfection apparatus to be visually accessible in operation, said door leaf is preferably at least partly formed with a transparent portion.

### Brief Description of the Drawings

The invention will in the following be described with reference to the accompanying drawings, which for the purpose of exemplification illustrate preferred embodiments of the invention.
Fig. 1 is a schematic view of a disinfection apparatus according to a first embodiment of the invention.
Fig. 2 is a perspective view of a door unit for the disinfection apparatus according to Fig. 1.
Figs 3a-3c illustrate the door unit for the disinfection apparatus according to Fig. 1 in a sequence where the door leaf is moved from an open position to a closed position.

### Description of Preferred Embodiments

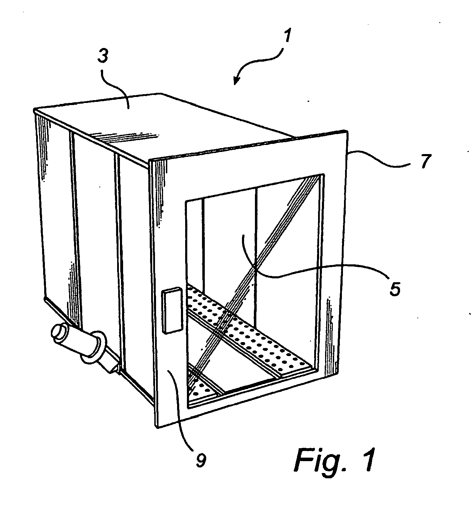

Fig. 1 shows a disinfection apparatus 1 according to a first embodiment of the invention, which has a housing 3 constituting a disinfection chamber 5 which is adapted to receive objects for disinfection. The chamber 5 consists partially of ceiling, floor and wall elements that can be assembled and are made of, for instance, stainless sheet steel. Moreover the disinfection apparatus comprises a door unit 7, which is adapted to close an entrance to the chamber 5.

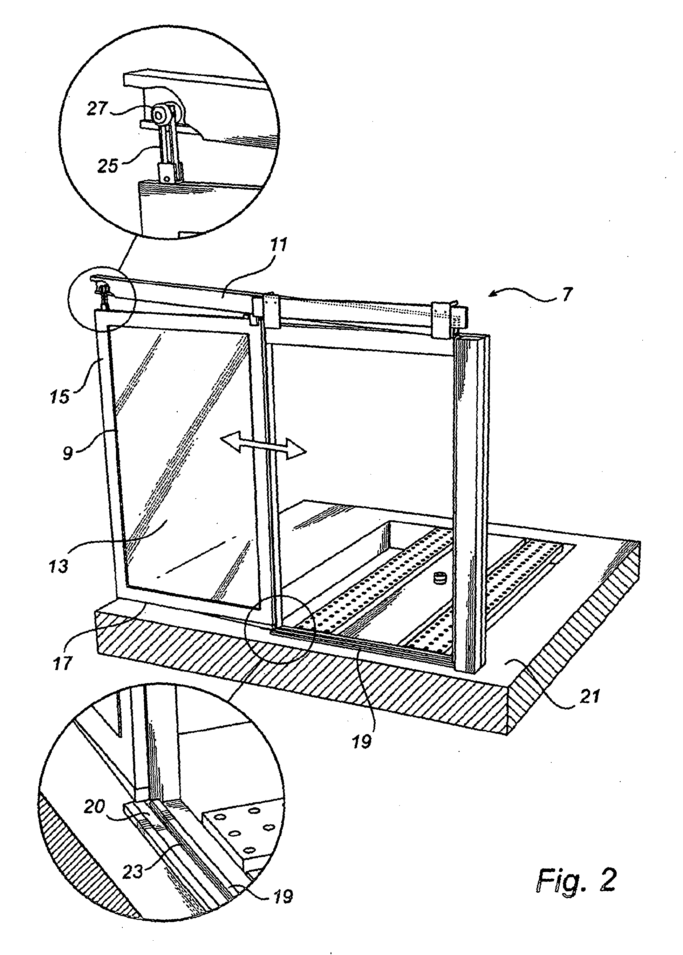

Fig. 2 shows parts of the door unit 7 of the disinfection apparatus, the door unit comprising a door leaf 9 for closing the disinfection chamber 5. The door leaf 9 is movably arranged on a guide element which extends above said door leaf 9. Moreover the door leaf 9 is in the embodiment shown in Fig. 2 provided with a transparent door portion 13 which is arranged in a frame structure 15 extending along the edges of the door portion, the frame structure preferably consisting of assembled sectional elements of, for instance, stainless sheet steel.

In the embodiment of the door unit 7 shown in Fig. 2, the lower edge 17 of the door leaf 9 is arranged to be held in a frame sealing portion 19 in a closed position of the door unit 7, the frame sealing portion 19 consisting in the shown embodiment of a threshold cavity 20 which is partially sunk into the floor 21 surrounding the disinfection apparatus. The frame sealing portion 19 is provided with a strip seal 23 which is arranged to sealingly engage the side of the door leaf 9 which is directed inwards to the chamber 5 in the closed position of the door unit 7.

Moreover the door frame is provided with seals which extend substantially along the periphery of the entrance to be moved into sealing engagement with the door leaf 9 in the closed position of the door unit 7. In a preferred embodiment of the disinfection apparatus 1, the door leaf 9 is arranged to be substantially horizontally moved into pressing engagement, in the closed position of the door unit, with seal strips arranged on the door frame by a force being applied to the door leaf 9 by operable power transmission means, which are not shown in the Figures. When the door unit 7 is to be opened, the force applied by the power transmission means to the door leaf 9 is removed, thereby allowing the door leaf 9 to be removed from the engagement with said seals which are arranged on the frame of the housing 3, thus making it possible to displace the door leaf 9 laterally without being braked in an unsatisfactory way by frictional forces from contact with said seals.

The guide element 11 which extends above the door leaf 9 is in a preferred embodiment arranged with an inclination α in the direction of motion of the door leaf 9 from the closed position to the open position, relative to a horizontal plane. The inclination α of the guide element 11 allows that the door leaf 9 is raised vertically relative to said lower frame sealing portion 19 as the door leaf 9 is moved from the closed position to the open position, and thus is lowered vertically as the door leaf 9 is moved from the open position to the closed position. The inclination α of the guide element 11 is preferably an angle between 1° and 10°, more preferred an angle between 1° and 5°, and in particular an angle between 2° and 3°.

As the door leaf 9 is raised vertically, when being moved from the closed position to the open position, the door leaf 9 is in a preferred embodiment allowed to be moved to a level at which its lower edge 17 is raised above the floor 21 surrounding the disinfection apparatus. By such a vertical raising of the door leaf 9, a door unit 7 is provided, where said threshold cavity 20 merely has to extend substantially in front of the entrance to the disinfection apparatus 1. It is thus possible to install a disinfection apparatus 1 with a sliding door, without requiring frequently extensive efforts to arrange a threshold cavity 20 in the motion of displacement of the entire door leaf 9.

Two sliding means 25 are arranged at the upper edge of the door leaf 9 to allow displaceable engagement with the guide element 11. Each sliding means 25 is provided with two rolls 27, which are held in the guide element 11 which preferably is formed as a square section with a through slot arranged on the side which is directed towards the door leaf.

In one embodiment of the disinfection apparatus 1 (not shown), the door leaf 9 is operated between the closed position and the open position, and vice versa, by a driving belt, to which the door leaf 9 is connected. Moreover the driving belt is preferably connected to a drive motor.

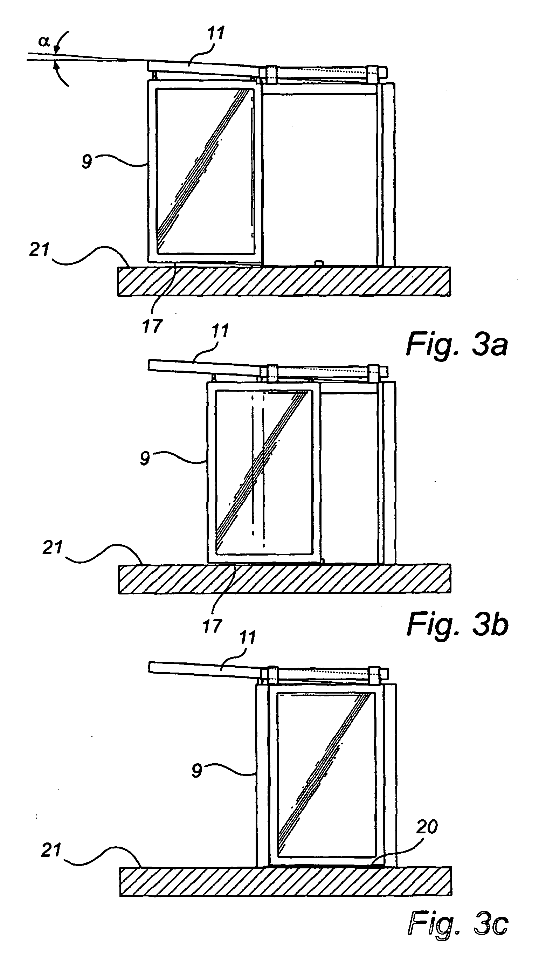

Figs 3a and 3b show how the door leaf 9 is moved from an open position to a closed position. In the open position according to Fig. 3a, the entire door leaf 9 is positioned above the floor 21 surrounding the disinfection apparatus 1. As the door leaf 9 is moved from the open position to the closed position, the door leaf 9 is lowered vertically by means of the guide element 11 inclined at the angle α. Thus, the vertical lowering of the door leaf 9 allows it to be gradually inserted into the threshold cavity 20 which is arranged in the lower part of the frame.

When the door leaf 9 has been moved all the way from the open position to the closed position according to Fig. 3c, the lower edge 17 of the door leaf 9 is received by the threshold cavity 20. It is also preferred for the door leaf 9 in the position shown in Fig. 3c to be moved into sealing engagement with the housing by the door leaf 9 being displaced substantially horizontally, which is not shown, towards seals which are arranged on the housing 3.

It will be appreciated that the above-described embodiment of the invention can be modified and varied by a person skilled in the art without departing from the inventive concept defined in the claims. For instance, it is possible to provide the vertical displacement of the door leaf 9 relative to the frame sealing portion 19 by the door leaf 9 first being displaced vertically upwards relative to the frame seal 19, so as then to be moved laterally relative to the disinfection chamber 5. Also the guide element 11 can be designed in various ways, where different portions have different inclinations. For instance, a first portion can be inclined in order to raise the door leaf 9 vertically while at the same time the door leaf 9 is displaced laterally, which inclined portion is followed by a horizontal portion for continued displacement of the door leaf 9 in the lateral direction. It is also possible to design the guide element 11 with a plurality of portions which are arranged with different inclinations relative to each other, to provide the vertical displacement of the door leaf. It will also be appreciated that the inclined portions of the guide element 11 can be designed as linearly inclined portions as well as curved portions.

It will further be appreciated that it is possible to arrange the above-mentioned strip seals on the door leaf 9, which constitute a seal between the housing 3 and the door leaf 9.

The lower frame seal 19 can also be arranged as a strip seal on the underside of the door leaf 9, which is directed towards the lower portion of the frame of the entrance to the chamber 5. Consequently it is possible to move, by the vertical displacement of the door leaf 9 when moved from the open position to the closed position, the strip seal into sealing engagement with the lower frame portion 19 of the chamber 5. However, it is possible to obtain a thresholdless entrance to the chamber 5 with a seal between the lower edge of the door leaf 9 and the lower frame portion 19 of the chamber 5 without arranging a threshold cavity 20.

## Claims

1. A disinfection apparatus (1) for disinfecting fluid cleaning of objects, such as health care objects, which disinfection apparatus (1) comprises
a housing (3) which forms a chamber (5) with an entrance having a frame,
a substantially laterally displaceable door leaf (9) which is arrangeable in a closed position to close said entrance, and
at least one guide element (11) which extends substantially in the direction of motion of the door leaf (9) and in whose direction of extension the door leaf (9) is displaceably arranged, wherein
a lower edge of the door leaf (9) constitutes a door sealing portion (17) which is arranged to sealingly cooperate with a corresponding lower sealing portion (19) of said frame in the closed position,
**characterised in that** the door sealing portion (17) of the door leaf (9) is arranged to be moved vertically relative to said lower frame sealing portion (19) as the door leaf (9) is moved from the closed position to an open position, and **in that** said at least one guide element (11) of the housing (3) extends above the door leaf (9), and the door leaf (9) is slidingly engaged with the guide element (11) by at least one sliding means (25), wherein said at least one sliding means (25) is arranged as a roll element.

2. A disinfection apparatus (1) as claimed in claim 1, wherein said guide element (11) at least partly is arranged with an inclined extent (α), in the direction of motion of the door leaf (9) from the closed position to the open position, in relation to a horizontal line.

3. A disinfection apparatus (1) as claimed in claim 1 or 2, wherein said lower frame sealing portion (19) is arranged as a part of a threshold cavity (20).

4. A disinfection apparatus (1) as claimed in claim 3, wherein said threshold cavity (20) is arranged in a groove in the floor (21) surrounding the disinfection apparatus (1).

5. A disinfection apparatus (1) as claimed in claim 3, wherein said threshold cavity (20) is arranged as a groove in at least a part of a threshold portion, which at least partly extends above the floor (21) surrounding the disinfection apparatus (1).

6. A disinfection apparatus (1) as claimed in any one of the preceding claims, wherein said door leaf (9) is moved into sealing cooperation with the housing (3) in the closed position by a substantially horizontal displacement of the door leaf (9) inwards to said chamber (5).

7. A disinfection apparatus (1) as claimed in any one of the preceding claims, wherein the guide element (11) is designed as an elongate square section with a slot arranged in the longitudinal direction thereof through a wall portion, said roll element comprising at least two rolls (27) which are adapted to run one on each side of said slot inside said section.

8. A disinfection apparatus (1) as claimed in any one of the preceding claims, wherein the door leaf (9) is connected to a driving belt to be slidably moved between the closed and the open position, and vice versa, which driving belt is driven by a motor.

9. A disinfection apparatus (1) as claimed in any one of claims 2-8, wherein the inclined extent (α) of said guide element (11) is an angle between 1° and 10°.

10. A disinfection apparatus (1) as claimed in any one of the preceding claims, wherein said door leaf (9) at least partly comprises a transparent portion (13).

## Patentansprüche

1. Desinfektionsvorrichtung (1) zum Desinfizieren von Gegenständen, wie etwa Gesundheitspflegegegenständen, mit einem Desinfektionsfluid, wobei die Desinfektionsvorrichtung (1) Folgendes umfasst:
ein Gehäuse (3), das eine Kammer (5) mit einem Eingang mit einem Rahmen bildet,
ein im Wesentlichen seitlich verschiebbares Türblatt (9), das in einer geschlossenen Stellung zum Verschließen des Eingangs angeordnet werden kann, und
zumindest ein Führungselement (11), das sich im Wesentlichen in der Bewegungsrichtung des Türblatts (9) erstreckt und in dessen Ausdehnungsrichtung das Türblatt (9) verschiebbar angeordnet ist, wobei
eine untere Kante des Türblatts (9) einen Türabdichtungsabschnitt (17) bildet, der angeordnet ist, um mit einem entsprechenden unteren Abdichtungsabschnitt (19) des Rahmens in der geschlossenen Stellung dichtend zusammenzuwirken,
**dadurch gekennzeichnet, dass** der Türabdichtungsabschnitt (17) des Türblatts (9) angeordnet ist, um in Bezug auf den unteren Rahmenabdichtungsabschnitt (19) vertikal bewegt zu werden, wenn das Türblatt (9) von der geschlossenen Stellung in eine offene Stellung bewegt wird, und dass sich das zumindest eine Führungselement (11) des Gehäuses (3) oberhalb des Türblatts (9) erstreckt und das Türblatt (9) durch zumindest ein gleitendes Mittel (25) mit dem Führungselement (11) gleitend in Eingriff ist, wobei das zumindest eine gleitende Mittel (25) als ein Rollelement angeordnet ist.

2. Desinfektionsvorrichtung (1) nach Anspruch 1 , wobei das Führungselement (11) zumindest teilweise mit einem in Bezug auf eine horizontale Linie geneigten Ausmaß (α) in der Bewegungsrichtung des Türblatts (9) von der geschlossenen Stellung in die offene Stellung angeordnet ist.

3. Desinfektionsvorrichtung (1) nach Anspruch 1 oder 2, wobei der untere Rahmenabdichtungsabschnitt (19) als ein Teil eines Schwellenhohlraums (20) angeordnet ist.

4. Desinfektionsvorrichtung (1) nach Anspruch 3, wobei der Schwellenhohlraum (20) in einer Rille im Boden (21), der die Desinfektionsvorrichtung (1) umgibt, angeordnet ist.

5. Desinfektionsvorrichtung (1) nach Anspruch 3, wobei der Schwellenhohlraum (20) als eine Rille in zumindest einem Teil eines Schwellenabschnitts angeordnet ist, der sich zumindest teilweise über dem Boden (21) erstreckt, der die Desinfektionsvorrichtung (1) umgibt.

6. Desinfektionsvorrichtung (1) nach einem der vorangegangenen Ansprüche, wobei das Türblatt (9) durch eine im Wesentlichen horizontale Verschiebung des Türblatts (9) nach innen zur Kammer (5) in die dichtende Zusammenwirkung mit dem Gehäuse (3) in der geschlossenen Stellung bewegt wird.

7. Desinfektionsvorrichtung (1) nach einem der vorangegangenen Ansprüche, wobei das Führungselement (11) als ein längliches Vierecksprofil mit einem in dessen Längsrichtung durch einen Wandabschnitt hindurch ausgebildeten Schlitz ausgelegt ist, wobei das Rollelement zumindest zwei Rollen (27) umfasst, die dazu angepasst sind, im Inneren des Profils an je einer Seite des Schlitzes zu laufen.

8. Desinfektionsvorrichtung (1) nach einem der vorangegangenen Ansprüche, wobei das Türblatt (9) mit einem Antriebsriemen verbunden ist, um gleitend zwischen der geschlossenen und der offenen Stellung und umgekehrt bewegt zu werden, wobei der Antriebsriemen von einem Motor angetrieben ist.

9. Desinfektionsvorrichtung (1) nach einem der Ansprüche 2 bis 8, wobei das geneigte Ausmaß (α) des Führungselements ein Winkel zwischen 1° und 10° ist.

10. Desinfektionsvorrichtung (1) nach einem der vorangegangenen Ansprüche, wobei das Türblatt (9) zumindest teilweise einen transparenten Abschnitt (13) umfasst.

## Revendications

1. Appareil de désinfection (1) pour désinfecter au liquide des objets comme des objets de soins médicaux, lequel appareil de désinfection (1) comprend
un boîtier (3) qui forme une chambre (5) comportant une entrée dotée d'un cadre,
un battant de porte (9) déplaçable sensiblement dans le sens latéral et qui peut être disposé dans une position fermée pour fermer ladite entrée, et
au moins un élément de guidage (11) qui s'étend sensiblement le sens de mouvement du battant de porte (9) et dans le sens d'extension duquel le battant (9) est disposé de manière déplaçable,
dans lequel un bord inférieur du battant de porte (9) constitue un segment d'étanchéité de porte (17) qui est disposé de manière à coopérer au niveau étanchéité avec un segment d'étanchéité inférieur correspondant (19) dudit cadre en position fermée,
**caractérisé en ce que** le segment d'étanchéité de porte (17) du battant de porte (9) est disposé de manière à se déplacer verticalement par rapport audit segment d'étanchéité de cadre inférieur (19) lorsque le battant de porte (9) est passé de la position fermée à une position ouverte et **en ce que** ledit au moins un élément de guidage (11) du boîtier (3) s'étend au dessus du battant de porte (9) et que le battant de porte (9) s'engage en glissant dans l'élément de guidage (11) grâce à au moins un moyen de glissement(25), ledit au moins un moyen de glissement (25) se présentant sous la forme d'un élément roulant.

2. Appareil de désinfection (1) selon la revendication 1, dans lequel ledit élément de guidage (11) est disposé au moins partiellement en extension inclinée (α) par rapport à une ligne horizontale dans le sens de mouvement du battant de porte (9) depuis la position fermée jusqu'à la position ouverte.

3. Appareil de désinfection (1) selon la revendication 1 ou 2, dans lequel ladite section d'étanchéité de cadre inférieur (19) est disposée sous forme d'une partie de cavité de seuil (20).

4. Appareil de désinfection (1) selon la revendication 3, dans lequel ladite cavité de seuil (20) est disposée dans une gorge du sol (21) entourant l'appareil de désinfection (1).

5. Appareil de désinfection (1) selon la revendication 3, dans lequel ladite cavité de seuil (20) est disposée dans une gorge dans au moins une partie d'un segment de sol qui s'étend au moins partiellement au dessus du sol (21) entourant l'appareil de désinfection (1).

6. Appareil de désinfection (1) selon l'une quelconque des revendications précédentes, dans lequel ledit battant de porte (9) est amené en coopération d'étanchéité avec le boîtier (3) en position fermée par un déplacement sensiblement horizontal du battant de porte (9) vers l'intérieur de ladite chambre (5).

7. Appareil de désinfection (1) selon l'une quelconque des revendications précédentes, dans lequel l'élément de guidage (11) est conçu sous forme d'une section carrée allongée comportant une fente pratiquée dans son sens longitudinal à travers un segment de paroi, ledit élément roulant comportant au moins deux roulettes (27) qui sont aptes à circuler chacune sur un côté de ladite fente à l'intérieur de ladite section.

8. Appareil de désinfection (1) selon l'une quelconque des revendications précédentes, dans lequel le battant de porte (9) est connecté à une courroie d'entraînement apte à se déplacer en glissant entre la position fermée et la position ouverte et vice versa, laquelle courroie d'entraînement étant entraînée par un moteur.

9. Appareil de désinfection (1) selon l'une quelconque des revendications 2 à 8, dans lequel l'extension inclinée (α) dudit élément de guidage (11) est un angle compris entre 1 ° et 10°.

10. Appareil de désinfection (1) selon l'une quelconque des revendications précédentes, dans lequel ledit battant de porte (9) comprend au moins partiellement une partie transparente (13).
